# EUROPEAN PATENT APPLICATION

(11) **EP 1 844 695 A1**
(43) Date of publication of application: **17.10.2007**
(21) Application number: 06711972.7
(22) Date of filing: 19.01.2006
(51) Int. Cl.: A61B 1/00

(54) **TREATMENT DEVICE FOR ENDOSCOPE**

(30) Priority: 31.01.2005 JP 2005023713
(71) Applicant: OLYMPUS MEDICAL SYSTEMS CORP., Shibuya-ku Tokyo 151-0072 (JP)
(72) Inventor: NAKAGAWA, Tsuyoshi, 1890025 (JP); YANUMA, Yutaka, Tokyo, 1860004 (JP)
(74) Representative: von Hellfeld, Axel
(86) International application number: PCT/JP2006/300726
(87) International publication number: WO 2006/080232

(57) **Abstract**

An endoscopic treatment instrument (1) includes: a cannular sheath section (24) having an entrance-opening section (36) at a base end section; and an elongated treatment instrument main body (37) having a basket (45) at a tip section, wherein the treatment instrument main body (37) inserted in the sheath section (24) via the entrance-opening section (36) is capable of advancing and retracting, the basket (45) can be advanced and retracted from the tip of the sheath section (24) by advancing and retracting the treatment instrument main body (37) with respect to the sheath section (24), the endoscopic treatment instrument (1) further includes an attachment section (30), disposed between the sheath section (24) and an endoscope (2) having a forceps port (21), for attaching the sheath section (24) to the endoscope (2) so that the entrance-opening section (36) and the forceps port (21) are disposed on a line so as to substantially face each other.

## Description

### Technical Field

The present invention relates to an endoscopic treatment instrument for use with an endoscope for conducting various treatments.
The present application is based on patent application No. 2005-023713 filed January 31, 2005, in Japan, the content of which is incorporated herein by reference.

### Background Art

Endoscopic treatment instruments have been used recently in medical fields for conducting various treatments while observing images obtained with an endoscope. Such a known endoscopic treatment instrument is configured to have an elongated treatment instrument main body that has a cannular sheath having an entrance-opening section provided on a base end section of the endoscopic treatment instrument and a basket provided on a tip section for enclosing a foreign body. A treatment instrument main body is inserted retractably through the sheath via an entrance-opening section so that the basket protrudes and recedes relative to the tip of the sheath by advancing and retracting the treatment instrument main body (see, for example, a Patent Document 1). By protruding from the basket from the tip of the sheath, it is allowed to expand in radial outward directions. On the other hand, by enclosing it into the sheath, it is allowed to contract in radial inward directions.

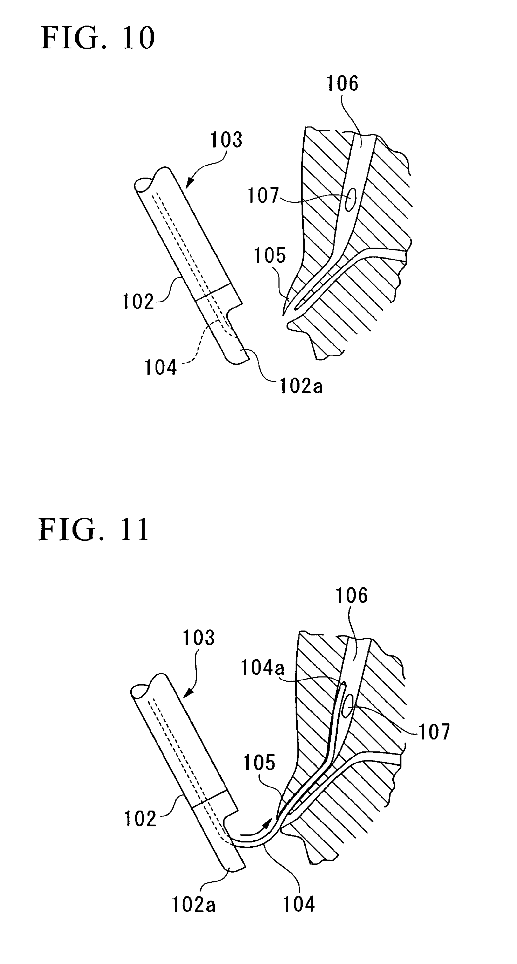

One of the treatments conducted by the endoscopic treatment instrument will be explained as follows with reference to an exemplary case for collecting a gall stone clogging a bile duct.
As illustrated in FIG. 10, an insertion section 102 of an endoscope 103 is first to be inserted and fed into a body cavity so that a tip section 102a of the insertion section 102 is disposed in the vicinity of a nipple 105. As illustrated in FIG. 11, a sheath 104 is fed from an operator's hand side of the endoscope 103 so as to extend from the tip section 102a. The sheath 104 is subsequently inserted in a bile duct 106. The sheath 104 is further passed forward through the bile duct 106 and stopped at a predetermined position where a tip section 104a is beyond a gall stone 107.

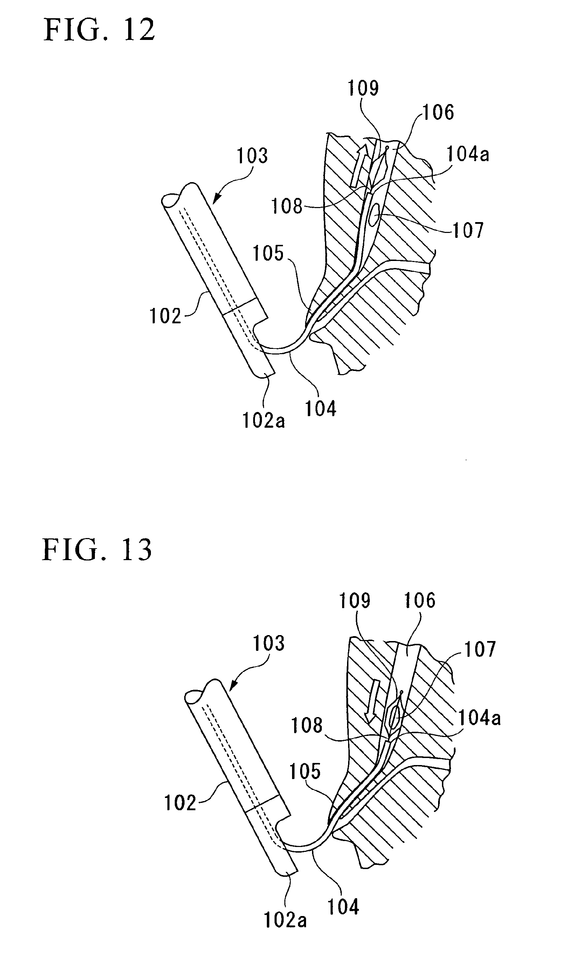

Forwarding further a treatment instrument main body 108 from here toward the sheath 104 while pausing the sheath 104 protrudes a basket 109 from the tip section 104a as illustrated in FIG. 12. Accordingly, the basket 109 expands into radial outward directions with respect to the sheath 104. After that, the basket 109 is retracted together with the sheath 104 to the position corresponding the gall stone 107 as illustrated in FIG. 13. Thus, the gall stone 107 is enclosed by the basket 109. Drawing the treatment instrument main body 108 while pausing the sheath 104 to retract the treatment instrument main body 108 relative to the sheath 104 allows the basket 109 to bend and contract in radial inward directions; thereby enclosing the basket 109 in the sheath 104.

The basket 109 in this state, i.e., enclosing the gall stone 107, is not enclosed in the sheath 104 fully. That is, the basket 109 grasps the gall stone 107 at a tip section 104a. Removing this state of the sheath 104 from the nipple 105 permits the gall stone 107 clogging the bile duct 106 to be extracted from the bile duct 106.

However, if a structure section develops in some point of the bile duct 106 and a gall stone 107 clogs the proximity of the structure in the bile duct 106, the gall stone 107 cannot be collected because a space allowing the basket 109 to protrude and expand from the tip section 104a of the sheath 104 cannot be obtained across the gall stone 107.

In this case, the operator draws the sheath 104, and a supporter simultaneously feeds the same drawn length of the treatment instrument main body 108. This provides movements of the sheath 104 and the treatment instrument main body 108 in opposite directions relative to each other, thereby retracting only the sheath 104 while substantially maintaining the basket 109 at a fixed position in the bile duct 106. The basket 109 can expand where the inserted tip section 104a has been previously disposed if there is not a sufficient space ahead of the tip section 104a.
[Patent Document 1] Japanese Unexamined Patent Application, First Publication No. 2000-5186

### DISCLOSURE OF INVENTION

### Problems to be solved by the Invention

However, collaborations by the operator and the supporter will meet significant difficulty in various treatments because the timings and the movements in retracting the sheath 104 and feeding the treatment instrument main body 108 must coincide between these people.

In view of the foregoing circumstances, it is an object of the present invention to provide an endoscopic treatment instrument that facilitates and prompts various treatments to handle various conditions in a body cavity without necessitating a collaboration by the endoscopist and the supporter therefor, i.e., by a standalone operation by the endoscopist.

### Means for solving the Problems

An endoscopic treatment instrument according to the present embodiment includes: a cannular sheath having an entrance-opening section at a base end section; and an elongated treatment instrument main body having a treatment section at a tip section, wherein the treatment instrument main body inserted in the sheath via the entrance-opening section is capable of advancing and recedeing, the treatment section can be advanced and retracted from the tip of the sheath by advancing and retracting the treatment instrument main body with respect to the sheath, and the endoscopic treatment instrument further includes an attachment section, disposed between the sheath and an endoscope having a forceps port, for attaching the sheath to the endoscope so that the entrance-opening section and the forceps port are disposed on a substantial line to face each other.

In the endoscopic treatment instrument according to the present invention, attaching the sheath to the endoscope via the attachment section places the entrance-opening section and the forceps port of the endoscope on substantially a line to face each other. Therefore, inserting the tip of the sheath in this state into the forceps port places the sheath in the vicinity of the forceps port and the treatment instrument main body inserted into the entrance-opening section on substantially the one line so that feed directions (forwarding directions) are opposite to each other. Grasping then the sheath and the treatment instrument main body disposed on substantially the one line and moving them together in the feeding directions of the treatment instrument main body provide simultaneous movements of drawing the sheath and feeding the treatment instrument main body. The movement of the drawn sheath is therefore equalized to the movement of the fed treatment instrument main body; thus, only the sheath retracts while the treatment section remains at a predetermined position.
This allows an easy standalone operation for an endoscopist to expand the treatment section at the position where the tip section has previously been disposed.

In the endoscopic treatment instrument according to the present invention, the attachment section should be preferably provided with an attachment adapter detachably attached to the endoscope; and an attachment member for detachably attaching the sheath to the attachment adapter.

In the endoscopic treatment instrument according to the present invention, the sheath is detachably attached to the endoscope via the attachment member and the attachment adapter. This state of the sheath is detachably attached to the attachment adapter.
This configuration permits the sheath to be attached to the endoscope reliably. In addition, the foregoing effect can be obtained in an existing apparatus having a new attachment adapter since an existing sheath and an endoscope do not have to be changed specifically.

### Effects of the Invention

The present invention provides appropriate, i.e., facilitated and prompt, operations of treatment instruments, without collaborations by the endoscopist and the supporter, based on various conditions in a body cavity. So if there is a stricture ahead of the point to be treated, the treatment section can expand where the tip section has been previously disposed.

### BRIEF DESCRIPTION OF DRAWINGS

FIG. 1 illustrates a first embodiment of the endoscopic treatment instrument in schematic view for combined use with an endoscope for conducting various treatments according to the present invention.
FIG. 2 is a schematic view illustrating the base end support section of FIG.1 disposed in an opposing position that provides a simultaneous movement of a flexible sheath and a pipe.
FIG. 3 illustrates the endoscopic treatment instrument of FIG. 1 in an enlarged side view
FIG 4 illustrates a basket protruding from the tip of a flexible sheath in schematic view.
FIG. 5 illustrates a gall stone enclosed in an expanded basket in schematic view.
FIG. 6 is a side view illustrating a modified example of the endoscopic treatment instrument of FIG. 1.
FIG. 7 illustrates a second embodiment of the endoscopic treatment instrument according to the present invention in side view.
FIG. 8 is a schematic view illustrating the base end support section of FIG. 7 disposed in an opposing position that provides a simultaneous movement of the flexible sheath and a slider.
FIG. 9 illustrates a modified example of the endoscopic treatment instrument of FIG. 7 in side view.
FIG. 10 is a schematic view illustrating the tip of an insertion section of an endoscope disposed in the vicinity of a nipple in a conventional treatment for collecting a gall stone clogging a bile duct.
FIG. 11 is a schematic view illustrating the tip of the sheath extended from the insertion section of the endoscope and inserted into the bile duct so as to be disposed across the gall stone in the conventional treatment for collecting the gall stone clogging the bile duct.
FIG. 12 is a schematic view illustrating the basket protruding and expanding from the tip of the sheath in the conventional treatment for collecting the gall stone clogging the bile duct.
FIG. 13 is a schematic view illustrating the gall stone enclosed in the basket in the conventional treatment for collecting a gall stone clogging in the bile duct.

### Explanation of Reference Numerals and Symbols

1: endoscopic treatment instrument
2: endoscope
9: forceps cap
24: sheath section (sheath)
25a: tip (tip of the sheath)
21: forceps opening section (forceps port)
26: base end support section (base end section)
30: attachment section
36: entrance-opening section
37: treatment instrument main body
45: basket (treatment section)

### BEST MODE FOR CARRYING OUT THE INVENTION

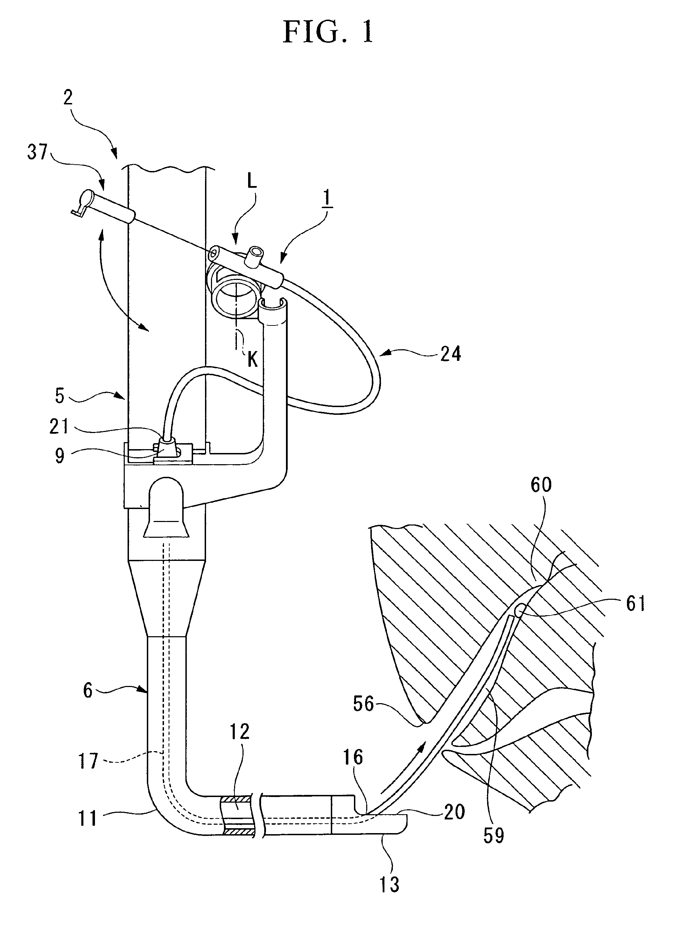

A first embodiment of the endoscopic treatment instrument according to the present invention will be explained with reference to FIGS. 1 to 6.
An endoscopic treatment instrument 1 according to the present embodiment is for combined use with an endoscope 2 for treating an affected part as illustrated in FIG. 1. The endoscope 2 will be explained first for combined use with the endoscopic treatment instrument 1.
The main components of the endoscope 2 are an endoscope-operating section 5 handled by an endoscopist for conducting various operations; and an endoscopic insertion section 6 to be inserted into a body cavity, e.g., a duodenum. That is, in this configuration of the endoscope 2, the endoscope-operating section 5 is connected to the end of the hollow elongated endoscopic insertion section 6 proximate to the endoscopist.

An endoscope system is constructed by appropriately combining the endoscope 2 with various external apparatuses not illustrated in the drawings, e.g., a light source apparatus, an image-processing apparatus, a monitor apparatus, an input keyboard, a suction pump apparatus, a water supply bottle. Usually, the foregoing external apparatuses are installed in shelves having a carrying apparatus. Some external apparatuses, e.g., the light source apparatus and the image-processing apparatus, are connected to the endoscope-operating section 5 via a universal cord (not shown in the drawings).

Provided on the endoscope-operating section 5 are operation levers and operation buttons (not shown in the drawings) for conducting various treatment operations. Provided on the tip of the endoscope-operating section 5 is a forceps cap 9 for insertion of the endoscopic treatment instrument 1. Formed in the forceps cap 9 is a forceps opening section (forceps port) 21 for the insertion of the endoscopic treatment instrument 1. The forceps opening section 21 communicates with a cannular channel 17 serving as a communication passage for the endoscopic treatment instrument 1.
In addition, the endoscopic insertion section 6 is provided with an elongated flexible tube section 11 having a base end section jointed with the endoscope-operating section 5; a bending section 12, provided to the tip of the flexible tube section 11, for bending the endoscopic insertion section 6; and a tip section provided on the tip of the bending section 12.

Formed on the outer periphery of the tip section is a recessing notched section 20 formed by cutting a part of the side. Formed on a part of a side of the notched section 20 is a channel exit opening section 16. The channel exit opening section 16 communicates with the forceps opening section 21 via the channel 17. Adjacently disposed beside the channel exit opening section 16 are an object lens of an observation optical system and an illumination lens of an illumination optical system. These optical systems are not illustrated in the drawings. Provided and protruding on a back wall of the notched section 20 is a nozzle, not illustrated in the drawings, that feeds air and water to clean the object lens and the illumination lens.

Concrete examples of the operation levers and the operation buttons provided on the endoscope-operating section 5 are a bending operation lever for providing horizontal and vertical movement to the bending section 12; an air-and-water-feeding button that provides air and liquid injection to the nozzle on the tip section; and a suction operation button for collecting mucus, etc. in the body cavity by providing a suction force to the channel exit opening section 16 via the channel 17.

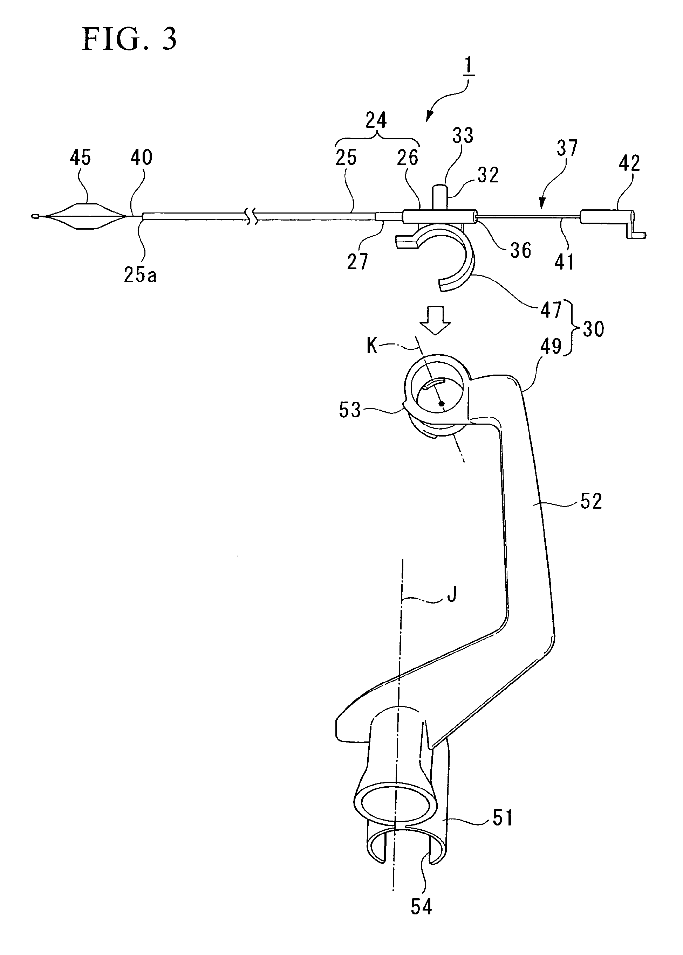

The endoscopic treatment instrument 1 according to the present invention will be explained next.
As illustrated in FIG. 3, provided to the endoscopic treatment instrument 1 are a cannular extending sheath section (sheath) 24; a treatment instrument main body 37 for conducting various treatments; and an attachment section 30 for attaching the sheath section 24 to the endoscope-operating section 5 as illustrated in FIG. 1.
The sheath section 24 is configured to comprise a flexible sheath 25 be inserted into the body cavity; a base end support section (base end section) 26 for supporting the flexible sheath 25; and a connection part 27 for jointing the flexible sheath 25 and the base end support section 26.

Provided to the base end support section 26 is a water-feed cock 32 inserted in the flexible sheath 25. A connection port section 33 is provided to the water-feed cock 32. Water can be fed to the flexible sheath 25 via the water-feed cock 32 by connecting a syringe, not illustrated in the drawings, to the connection port section 33. Provided on the base end of the base end support section 26 is an entrance-opening section 36 communicating with the flexible sheath 25. In addition, provided to the base end support section 26 is a hook member 47 having a hook shape.

Furthermore, the treatment instrument main body 37 is provided with a flexible operation wire 40 and a solid pipe 41 that is concentrically jointed to the operation wire 40.
Provided to the tip of the operation wire 40 is a basket (treatment section) 45 formed by a plurality of flexible wires. The basket 45, formed in a basket shape, expands and contracts in radial directions with respect to the operation wire 40. The expanded basket 45 captures a foreign body into the basket 45, and the contraction of the basket 45 encloses this state of the foreign body.

Turning a handler section 42, provided to a base end of the pipe 41, around an axial line of the operation wire 40 and the pipe 41 rotates the basket 45 via the pipe 41 and the operation wire 40.
Contracting and inserting the basket 45 into the entrance-opening section 36 in this configuration permits the sheath section 24 to support the operation wire 40 and the pipe 41 retractably. Grasping the pipe 41 or the handler section 42 and advancing and retracting it provides the extension and the retraction of the basket 45 relative to the tip (the tip of the sheath) 25a of the flexible sheath 25. Accordingly, the basket 45 having protruded from the tip 25a expands, and the basket 45 contracts while being enclosed into the flexible sheath 25.

Provided to the attachment section 30 are a hook member 47 and an attachment adapter 49 detachably attached to the endoscope-operating section 5 illustrated in FIG. 1.
The attachment adapter 49 is provided with a main attachment section 51, having a C-letter cross-sectional view, having a notched section 54 on a part of the outer periphery. Extending from the main attachment section 51 is an arm section 52. A cylindrical cylindrical-attachment section 53 is formed on a tip of the arm section 52. The main attachment section 51 and the cylindrical-attachment section 53 are disposed so that a center axial line J of the main attachment section 51 is orthogonal to a center axial line K of the cylindrical-attachment section 53. In addition, the center axial line J is disposed so that the attachment adapter 49 attached to the endoscope-operating section 5 is parallel with the opening direction of the forceps opening section 21.

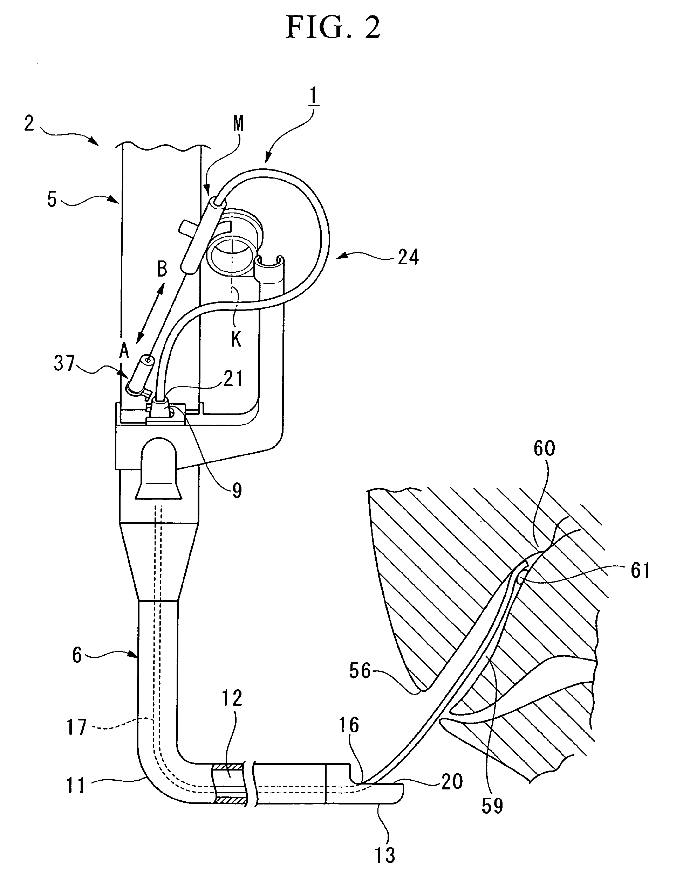

Compressing the main attachment section 51 in this configuration while contacting the main attachment section 51 to the endoscope-operating section 5 deforms the main attachment section 51, thereby fitting the main attachment section 51 to the endoscope-operating section 5 via the notched section 54. Compressing the hook member 47 while contacting the hook member 47 to the cylindrical-attachment section 53 deforms the hook member 47, thereby fitting the hook member 47 to the cylindrical-attachment section 53. This attaches the sheath section 24 to the attachment adapter 49 detachably, thereby rotatably supporting the sheath section 24 in this state around the center axial line K. That is, attaching the sheath section 24 to the endoscope-operating section 5 via the attachment adapter 49 allows the base end support section 26 to rotate around the center axial line K between an orthogonal position L where the forceps opening section 21 is disposed to be substantially orthogonal to the entrance-opening section 36 as illustrated in FIG. 1 and an opposing position M where the forceps opening section 21 is disposed to be on substantially a line with the entrance-opening section 36 as illustrated in FIG. 2.

A method for using the endoscopic treatment instrument 1 having the foregoing configuration according to the present embodiment will be explained next with reference to a case in which the foregoing gall stone is collected.
Suppose a structure section 60 formed in a bile duct 59 blocks the passageway, and a gall stone 61 clogs the proximate front of the structure section 60 in the present embodiment as illustrated in FIG. 1.
The sheath section 24 is attached to the endoscope-operating section 5 illustrated in FIG. 1 via the attachment adapter 49. The base end support section 26 is consequently disposed at the orthogonal position L. The tip 25a is inserted into the forceps opening section 21 as illustrated in FIG. 1 while enclosing the basket 45 in the flexible sheath 25. The flexible sheath 25 grasped by fingers is fed toward the tip section of the endoscopic insertion section 6. The flexible sheath 25 consequently advances in the channel 17. The feed is stopped at a point where the tip 25a is disposed in the tip section.

The tip section is disposed in the vicinity of a nipple 56 by grasping the endoscope-operating section 5 and inserting the endoscopic insertion section 6 into the body cavity while illuminating illumination light and observing images obtained through the object lens. The flexible sheath 25, extending from the forceps opening section 21, grasped by the fingers is fed while maintaining the foregoing dispositions. The tip 25a of the flexible sheath 25 consequently protrudes outward from the channel exit opening section 16. The flexible sheath 25 is fed and extended from the tip section 13. The flexible sheath 25 is subsequently inserted into a bile duct 59 to extend along the bile duct 59. The extension of the flexible sheath 25 is stopped at a predetermined position where the tip 25a is beyond a gall stone 61.

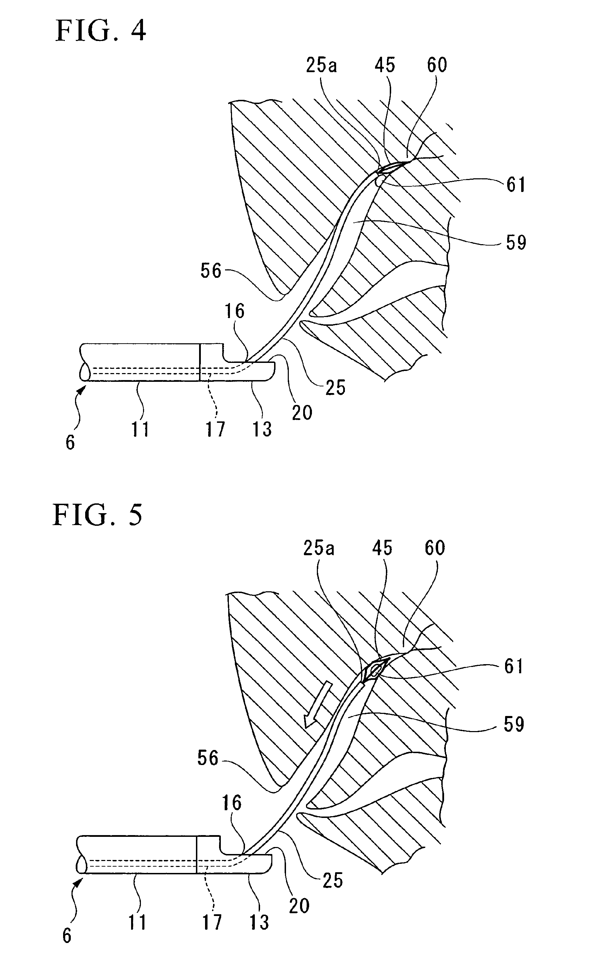

The base end support section 26 is rotated from this position to be disposed at the opposing position M illustrated in FIG. 2. The forceps opening section 21 and the entrance-opening section 36 are consequently disposed to face each other on substantially one line. The flexible sheath 25 extending outward from the forceps opening section 21 and the pipe 41 extending outward from the entrance-opening section 36 are therefore disposed on substantially one line so that feed directions are opposite. The flexible sheath 25 and the pipe 41 disposed on substantially the one line are grasped by the fingers and moved together in the feed direction B of the pipe 41. Accordingly, the pipe 41 moves in the feed direction B forwards toward the flexible sheath 25. On the other hand, the flexible sheath 25 retracts toward the pipe 41 since the flexible sheath 25 is drawn in the feed direction B of the pipe 41, i.e., the direction opposite to the feed direction A of the flexible sheath 25. This results in the movement of the drawn flexible sheath 25 being equal to the movement of the fed pipe 41, thereby retracting only the flexible sheath 25 while the operation wire 40 and the pipe 41 are paused in the endoscope 2. The basket 45 appearing outward expands at the position where the tip 25a has been previously disposed as illustrated in FIG. 4 since the nipple 56 and the tip-opening section 16 of the endoscope 2 do not move during a treatment operation.

After that, the basket 45 is retracted together with the pipe 41 to the position corresponding to the gall stone 61 as illustrated in FIG. 5. Thus, the gall stone 61 is enclosed by the basket 45. In a case where the gall stone 61 is hardly enclosed in the basket 45, the basket 45 is turned with the handler section 42 or moved back-and-forth to accommodate the gall stone 61 appropriately in the basket 45. The gall stone 61 accomodated in the basket 45 can be grasped by the basket 45 and the tip 25a by drawing the pipe 41 while pausing the flexible sheath 25, and by retracting the pipe 41 and the operation wire 40 with respect to the flexible sheath 25. The gall stone 61 can be extracted from the bile duct 59 by removing the flexible sheath 25 from the nipple 56 similarly to the conventional case.

In view of the foregoing, the endoscopic treatment instrument 1 according to the present embodiment allows the flexible sheath 25 and the operation wire 40 disposed on substantially the one line to be moved together by grasping them with fingers since the flexible sheath 25 and the pipe 41 are disposed on substantially the one line while the feed directions are opposite to each other when the sheath section 24 is attached to the endoscope-operating section 5 via the attachment adapter 49, and the base end support section 26 is disposed at the opposing position M. This equalizes the drawing movement of the flexible sheath 25 to the feeding movement of the pipe 41, thereby allowing the basket 45 to protrude at the position where the tip 25a has been previously disposed. This configuration provides appropriate standalone operations for an endoscopist that facilitates and prompts various treatments to handle various conditions in a body cavity without necessitating a collaboration by the endoscopist and the supporter therefor.

Also, the attachment adapter 49 permits the sheath section 24 to be attached to the endoscope-operating section 5 reliably. In addition, the foregoing effect can be obtained in an existing apparatus having a new attachment adapter 49 since an existing sheath and an endoscope do not have to be changed specifically.
Furthermore, the sheath section 24 attached to the arm section 52 rotatably via the hook member 47 and the cylindrical-attachment section 53 easily advances and retracts between the orthogonal position L and the opposing position M, therefore, various treatments can be facilitated more significantly.

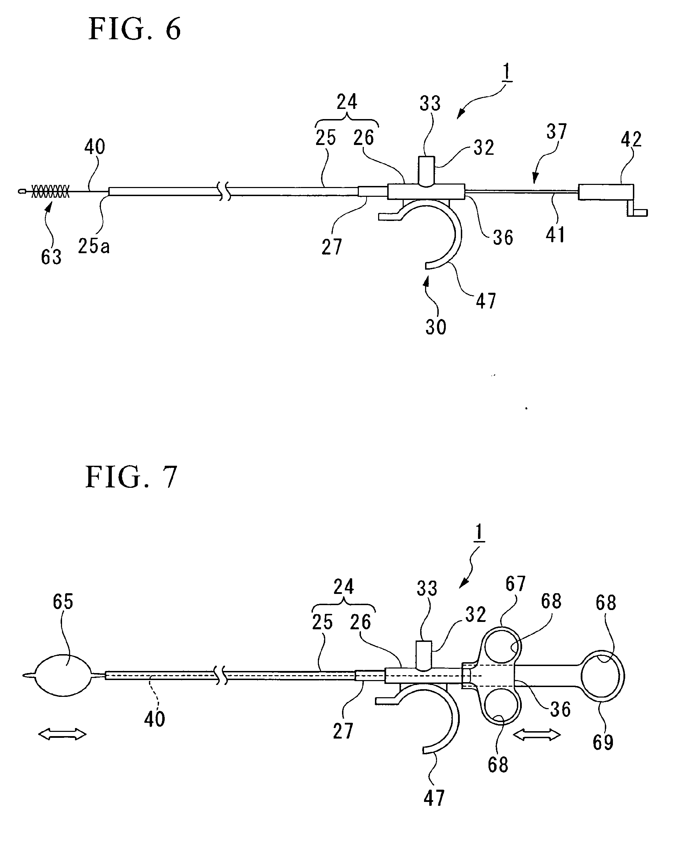

Note that the attachment adapter 49 may not be provided in the present embodiment although the attachment section 30 is provided with the attachment adapter 49 according to the foregoing description. In this case, an arm may be extended from the base end support section 26, and a hook member 47 may be disposed at the tip of the arm to fit to the endoscope-operating section 5.
Furthermore, the present invention is not limited to the configuration in which the basket 45 is a treatment section, i.e., the configuration may be appropriately modified according to various treatments. For example, a brush 63 may be disposed for removing cells in the body cavity as illustrated in FIG. 6.
Note that is not limited to the configuration in which the tip 25a is fed across the gall stone 61 to expand the basket 45, i.e., the tip 25a may be fed to the position orthogonally adjacent to the gall stone 61 with respect to the advancing direction of the tip 25a, and the basket 45 may expand at that position. This allows the basket 45 to expand adjacent to the gall stone 61 and accommodate the gall stone 61 if the space beyond the gall stone 61 is too narrow.

A second embodiment of the endoscopic treatment instrument according to the present invention will be explained next with reference to FIGS. 7 to 8. The same reference numerals are added to the elements illustrated in FIGS. 7 to 8 that are the same as those illustrated in FIGS. 1 to 6 so as to omit the duplicate explanation.
The fundamental configuration of the present embodiment is the same as that of the first embodiment; the difference is as follows. That is, the treatment section used in the endoscopic treatment instrument 1 of the present embodiment is a snare 65 having a flexible wire loop disposed on the tip of the operation wire 40 as illustrated in FIG. 7. In addition, provided on the base end of the base end support section 26 is a base bore section 67 extending in the direction orthogonal to the axial line of the flexible sheath 25. Formed on both ends in the longitudinal direction of the base bore section 67 are insertion holes 68. Also, an entrance-opening section 36 communicating with the flexible sheath 25 is formed on the base bore section 67. An extended bar slider 69 concentrically connected to the operation wire 40 is retractably inserted in the entrance-opening section 36. Formed at the rear end of the slider 69 is an insertion hole 68.

The endoscopic treatment instrument 1 is grasped by fingers inserted in each insertion hole 68. Advancing and retracting the slider 69 in the axial direction in this configuration allows the snare 65 to protrude and retract with respect to the tip 25a of the flexible sheath 25 via the operation wire 40. The snare 65 having protruded from the tip 25a expands in radial directions.

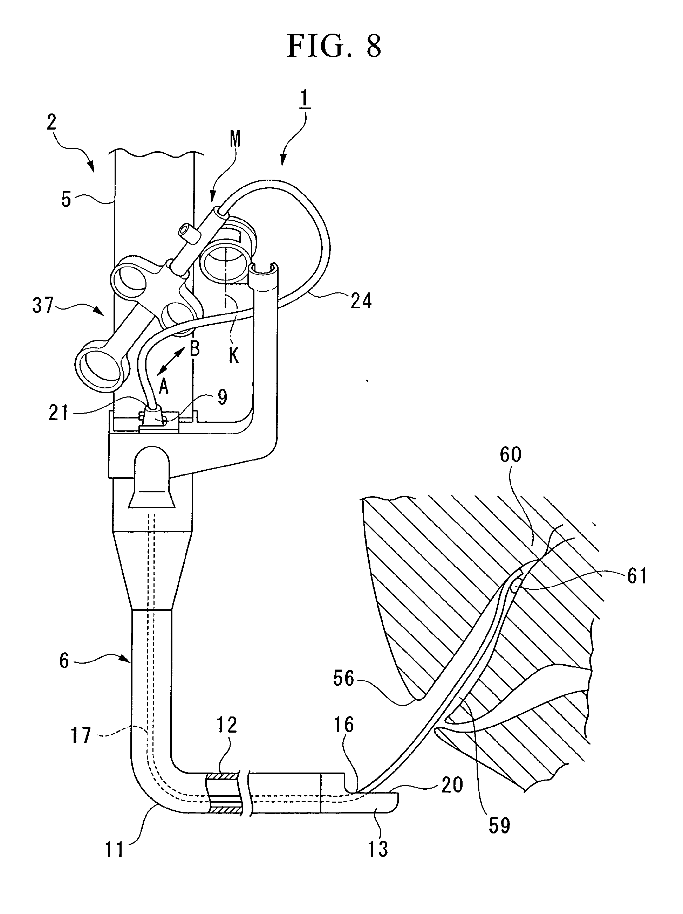

Furthermore, as illustrated in FIG. 8, attaching the sheath section 24 to the endoscope-operating section 5 via the attachment adapter 49 and disposing the base end support section 26 at the opposing position M place the flexible sheath 25 and the slider 69 on substantially one line so that the feed directions are opposite to each other. Therefore, the flexible sheath 25 and the slider 69 disposed on substantially the one line and grasped by fingers can be moved together.
This configuration provides effects similar to those obtained in the foregoing first embodiment.

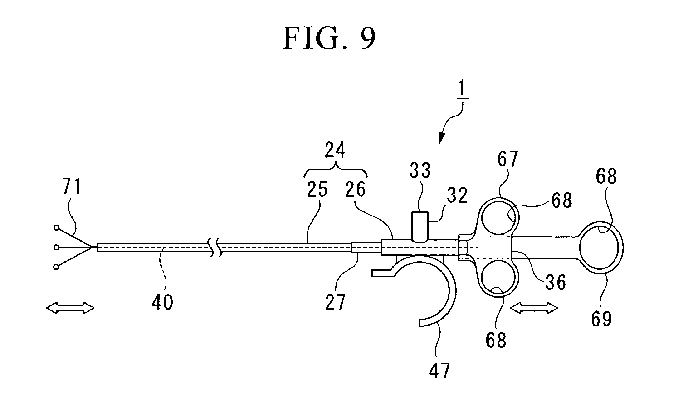

Furthermore, the present invention is not limited to the configuration using the snare 65 as a treatment section, i.e., the configuration may be appropriately modified according to various treatments. For example, a fork-shaped grasping section 71 may be provided as illustrated in FIG. 9 showing a configuration in which a plurality of flexible wires expand gradually from the base end to the tip.
Although the present invention has been described with respect to its preferred embodiments, the present invention is not limited to the embodiments described above. The configuration of the present invention allows for addition, omission, substitution and further modification without departing from the spirit and scope of the present invention. The present invention is not limited to the above descriptions but is limited only by the appended claims.

### Industrial Applicability

The present invention relates to an endoscopic treatment instrument comprising: a cannular sheath having an entrance-opening section at a base end section; and an elongated treatment instrument main body having a treatment section at a tip section, wherein the treatment instrument main body inserted in the sheath via the entrance-opening section is capable of advancing and receding, the treatment section can be advanced and retracted from the tip of the sheath by advancing and retracting the treatment instrument main body with respect to the sheath, and the endoscopic treatment instrument further comprises an attachment section, disposed between the sheath and an endoscope having a forceps port, for attaching the sheath to the endoscope so that the entrance-opening section and the forceps port are disposed on a substantial line to face each other. The endoscopic treatment instrument according to the present invention provides appropriate, i.e., facilitated and prompt, operations of treatment instruments, without collaborations by the endoscopist and the supporter, based on various conditions in a body cavity. So if there is a structure ahead of the point to be treated, the treatment section can expand where the tip section has been previously disposed.

## Claims

1. An endoscopic treatment instrument comprising:
a cannular sheath having an entrance-opening section at a base end section; and
an elongated treatment instrument main body having a treatment section at a tip section, wherein
the treatment instrument main body inserted in the sheath via the entrance-opening section is capable of advancing and retracting,
the treatment section can be advanced and rtracted from the tip of the sheath by advancing and retracting the treatment instrument main body with respect to the sheath, and
the endoscopic treatment instrument further comprises an attachment section, disposed between the sheath and an endoscope having a forceps port, for attaching the sheath to the endoscope so that the entrance-opening section and the forceps port are disposed on a line so as to substantially face each other.

2. The endoscopic treatment instrument according to Claim 1, wherein
the attachment section is provided with an attachment adapter detachably attached to the endoscope; and an attachment member provided to the sheath to detachably attach the sheath to the attachment adapter.
